# EUROPEAN PATENT APPLICATION

(11) **EP 3 708 071 A1**
(43) Date of publication of application: **16.09.2020**
(21) Application number: 19163190.2
(22) Date of filing: 15.03.2019
(51) Int. Cl.: A61B 5/024, A61B 5/0245, A61B 5/08, A61B 5/00

(54) **DEVICE, SYSTEM, METHOD AND COMPUTER PROGRAM FOR DETECTING ATRIAL FIBRILLATION**

(71) Applicant: Koninklijke Philips N.V., 5656 AG Eindhoven (NL)
(72) Inventor: BONOMI, Alberto Giovanni, 5656 AE Eindhoven (NL); FERREIRA DOS SANTOS DA FONSECA, Pedro Miguel, 5656 AE Eindhoven (NL)
(74) Representative: de Haan, Poul Erik

(57) **Abstract**

One problem in atrial fibrillation detection is that respiratory cycles induce a variation in the inter-beat interval (IBI) signal (140) determined from a cardiac activity signal (130). This results in a large number of false detected episodes. Analyzing the variability and/or chaotic structure of an IBI signal (140) as well as the frequency pattern of said IBI signal (140), i.e., the respiratory frequency power (180), e.g., in the power spectral density (PSD) spectrum (160) of said IBI signal (140), allows reducing the number of false detected episodes of atrial fibrillation.

## Description

### FIELD OF THE INVENTION

The present invention relates to a device, system, method and computer program for detecting atrial fibrillation.

### BACKGROUND OF THE INVENTION

The most common type of cardiac arrhythmia is atrial fibrillation. Atrial fibrillation is a pathological cardiac condition affecting millions of patients and is particularly prevalent for older patients. It leads to a severe increase of risk for an embolic stroke and heart failure.

Early detection of atrial fibrillation is problematic because of its paroxysmal nature and because the abnormal heart rhythm is often asymptotic with vague non-specific symptoms. Paroxysmal means in that context that the condition suddenly appears and the event autonomously stops. Hence, in order to properly capture atrial fibrillation events long-term monitoring of cardiac activities is essential, especially in the early stages of development.

Current atrial fibrillation monitoring technology is based on obtrusive and costly devices such as Holter monitors or implantable loop recorders. Holter can collect high-quality (multiple-lead) information on the patient electrocardiogram (ECG) so that the trained technician can provide a highly accurate interpretation of the patient's heart rhythm. A clear disadvantage of these systems is that they are quite obtrusive and the typical monitoring duration is 24 to 48 hours only. Thus, many atrial fibrillation patients are missed. An alternative solution for improving atrial fibrillation detection represent implantable loop recorders, which are capable of tracking ECG and the patient heart rhythm for very long time (up to a few years). As a disadvantage, they carry high costs and patients need to undergo surgery procedures.

Emerging digital health and wearable technologies are opening new avenues to allow for user-acceptable and non-stigmatizing long-term cardiac monitoring. In the last few years several commercial wearable devices have been developed that use plethysmography (PPG) sensors to continuously measure heart rate in free-living conditions. Similarly, innovative bio-sensors attached to the chest of a patient have been developed to conveniently measure electrocardiogram (ECG) for prolonged time periods. These tools can be used to accurately detect irregularities in the heart beat sequence and to identify episodes of atrial fibrillation. They represent convenient and low-cost solutions to support long-term monitoring of atrial fibrillation of individuals at risk of severe health events (stroke, heart failure deterioration, etc.) due to arrhythmia.

A constant problem in atrial fibrillation detection is the large number of false detected episodes. These episodes may occur due to natural and benign forms of arrhythmia such as those related to respiration. Respiratory cycles induce a variation in the inter-beat-interval (IBI) pattern that in some cases can be confused with atrial fibrillation, wherein IBIs are the time intervals between individual beats of the heart. During the so-called respiratory sinus arrhythmia (RSA), the IBI is modulated by changes in pulmonary blood pressure in response to cycles of chest expansion and relaxation. Such phenomenon is particularly pronounced during deep inhalation and exhalation, which can occur in different normal conditions for instance during slow-wave sleep, or during wakeful periods of relaxed, slow-paced breathing. A method or device capable of long-term monitoring of hearth rhythm for atrial fibrillation detection should be capable of distinguishing the IBI variability due to pronounced RSA from that due to atrial fibrillation.

US 2018/279891 A1 discloses a method for detection of atrial fibrillation based on IBI and PPG morphology features. However, there remains a need to decrease further the probability of false atrial fibrillation detection, e.g. due to artifacts or noises.

### SUMMARY OF THE INVENTION

It is an object of the present invention to provide a device, system, method and computer program for detecting atrial fibrillation of a subject with a reduced number of false atrial fibrillation detections.

In a first aspect of the present invention a device is presented that comprises a processing unit configured to
obtain a cardiac activity signal of the subject,
determine an inter-beat interval, IBI, signal representing the IBIs between heart beats in said cardiac activity signal,
estimate the presence of atrial fibrillation of the subject based on the IBI signal,
obtain the respiratory frequency of the subject,
determine the respiratory frequency power at said respiratory frequency, and
detect atrial fibrillation of the subject based on the determined respiratory frequency power and the estimated presence of atrial fibrillation.

According to another aspect a system is provided comprising:
a sensor configured to acquire a cardiac activity signal of the subject; and
a device according to the present invention for detecting atrial fibrillation of a subject based on the acquired cardiac activity signal of the subject.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed device, in particular as defined in the dependent claims and as disclosed herein.

The present invention solves a returning issue of atrial fibrillation detection, which is the large number of falsely detected episodes of atrial fibrillation that may occur due to natural and benign forms of arrhythmia such as those related to respiration. Information regarding certain aspects of the respiratory effort of a subject, for example the respiratory frequency and respiratory depth, can be deduced from the obtained cardiac activity signal of the subject.

The respiratory cycles induce a variation in the IBI signal that in some cases can be confused with atrial fibrillation. It should be noted that even for a normal heart function each IBI differs from beat to beat, but the irregularity and chaotic pattern may be more or less pronounced due to the influence of respiration. For one beat the IBI may, e.g., be 0.8 seconds, for the next beat 0.85 seconds, for the next beat 0.82 seconds, etc. The IBI signal according to the present invention represents the IBIs between heartbeats in the cardiac activity signal. Hence, according to said example the IBI of 0.8 seconds, 0.85 seconds, 0.82 seconds, etc. are stored in the IBI signal. Analyzing the IBI signal irregularity with measures of respiration-induced variability in IBI enables to improve detection of atrial fibrillation and reject falsely detected episodes of atrial fibrillation during prolonged periods of deep inhale and exhale that may occur for example during sleep.

An element to measure the respiration-induced irregularity and to overcome the problem of false detection of atrial fibrillation is to determine the respiratory frequency power at the respiratory frequency in a power spectral density (PSD) spectrum of the IBI signal. Alternative options to extract the respiratory information (i.e., respiratory frequency and respiratory frequency power) are the analysis of PPG pulse envelope modulation, R-peak amplitude modulation in an ECG signal, bio-impedance modulation or analysis of chest movement using accelerometers. Thus, in general the first step may be to obtain the respiratory frequency of the subject.

According to an embodiment the respiratory frequency of the subject may be received or retrieved from an external sensor, but the respiratory frequency may also be directly derived from the computed PSD spectrum of the IBI signal. The PSD of the IBI signal describes the distribution of power into frequency components composing the signal and allows obtaining the respiration frequency. Typically the respiration frequency corresponds to a local maximum in the PSD spectrum in the frequency range from 0.1 to 0.5 Hz, in particular from 0.15 to 0.4 Hz referring to the typical breathing rate of a person.

According to an embodiment, the processing unit is configured to derive the respiratory frequency from said PSD spectrum by identifying the local maximum in said frequency range. A high power of the local maximum refers to a strong influence of respiration. As the atrial fibrillation of the subject is detected based on the determined respiratory frequency power, a high power of the local maximum at the respiratory frequency may be a strong hint that there is, in fact, no atrial fibrillation, even if atrial fibrillation has been estimated based on the IBI signal.

According to another embodiment, the processing unit is configured to determine the IBI signal by detecting local minima and/or maxima in the cardiac activity signal. A cardiac activity signal may, e.g., be a PPG or ECG signal. The latter signal typically comprises P waves, R waves and T waves representing local minima and/or maxima in the cardiac activity signal. The IBI signal is determined by detecting these local minima and/or maxima and calculating the time periods between different maxima and/or minima, such as the time period between the maxima of two adjacent R waves.

According to another embodiment, the processing unit is configured to estimate the presence of atrial fibrillation by computing an initial probability of the presence of atrial fibrillation from the IBI signal. Preferably, the initial probability is computed from the IBI signal by use of a Markov model or based on the variability and/or chaotic structure in the IBI signal. A Markov model is a stochastic model used to model randomly changing systems and enables to assess the probability that a certain IBI sequence in the IBI signal is generated by a process typical of atrial fibrillation (see, e.g., A. G. Bonomi et al.: Atrial Fibrillation Detection Using a Novel Cardiac Ambulatory Monitor Based on PhotoPlethysmography at the Wrist, Journal of the American Heart Association, 7(15), (2018)). Alternatively, features of the IBI signal regarding normalized variability, such as root mean square of the successive differences (RMSSD), normalized absolute deviation (NADev) or normalized absolute difference (NADiff), and/or features regarding entropy, such as sample entropy, Shannon entropy and/or coefficients of the sample entropy may be computed from the IBI signal.

Alternatively, a machine learning algorithm may be used by the processing unit to combine the computed variability and/or entropy with a machine learning algorithm, e.g., support vector machine or logistic regression model to discriminate atrial fibrillation from other types of heart rhythm.

According to another embodiment, the processing unit is configured to detect atrial fibrillation by determining a final probability of the presence of atrial fibrillation from the determined respiratory frequency power and estimated presence of atrial fibrillation. As already explained above, according to embodiments an initial probability of the presence of atrial fibrillation may be computed from the IBI signal. If the IBI signal shows a strong irregularity and chaotic structure, the initial probability would be high. If, however, on the other hand, the respiratory frequency power is large, the final probability of atrial fibrillation would be reduced, i.e., at least be lower than the initial probability. Accordingly, the final probability would be larger than the initial probability if the respiratory frequency power is extremely low, which indicates that the respiratory has only marginal influence on the hearth rhythm. Thus, the analysis of the variability and/or chaotic structure in the IBI signal attributable to respiration enables to re-adjust the initial probability to obtain a more accurate final probability, which has significantly less influence of the respiration than the initial probability.

According to another embodiment, the final probability of the presence of atrial fibrillation allows the processing unit to generate a binary decision of the detection or absence of atrial fibrillation, in particular by comparing the initial probability with the final probability. If, e.g., the final probability is much lower than the initial probability, a strong influence of respiration may have caused the large initial probability. In this case, the binary decision would be 'no' referring to no detected atrial fibrillation. Preferably, the final probability may further be compared to predetermined probabilities, such as 50 %. If the final probability is larger than 50 %, atrial fibrillation is detected. If the final probability is smaller than 50 %, no atrial fibrillation is detected. Steps in between and various increments to classify the decision may be implemented.

The sensor for acquiring a cardiac activity signal of the subject may be a photoplethysmography (PPG) sensor, electrocardiography (ECG) sensor, ballistocardiography (BCG) sensor or another wearable sensor configured to continuously record cardiac activity signals of the subject.

The system may further comprise another sensor configured to measure the respiration rate of the subject, such as an accelerometer, impedance sensor, resistive respiration belt, ballistocardiography sensor or a seismocardiography sensor. In the case of seismocardiography or ballistocardiography the sensor is conventionally configured such that it responds to accelerations orthogonal to the surface of the thorax and is well suited to measure respiratory movements from which respiratory frequency can be easily measured.

According to another embodiment, the sensor configured to acquire a cardiac activity signal of the subject and the sensor configured to measure the respiration rate of the subject are the same.

### BRIEF DESCRIPTION OF THE DRAWINGS

These and other aspects of the invention will be apparent from and elucidated with reference to the embodiment(s) described hereinafter. In the following drawings
Fig. 1 shows a flowchart illustrating a system and a device for detecting atrial fibrillation according to the present invention.
Fig. 2 shows a flowchart illustrating a method for detecting atrial fibrillation according to the present invention.
Fig. 3 illustrates an exemplary signal waveform of an ECG signal.
Fig. 4 illustrates an exemplary signal waveform of a PPG signal and a corresponding IBI signal during atrial fibrillation.
Fig. 5 shows a diagram of an example of signals used in the detection of false atrial fibrillation.
Fig. 6 shows a diagram of another example of signals used in the detection of false atrial fibrillation.
Fig. 7 shows a diagram showing a computed PSD spectrum of an IBI signal for different awake and sleep phases of a subject.
Fig. 8 shows a diagram of another example of signals used in the detection of correct atrial fibrillation.

### DETAILED DESCRIPTION OF EMBODIMENTS

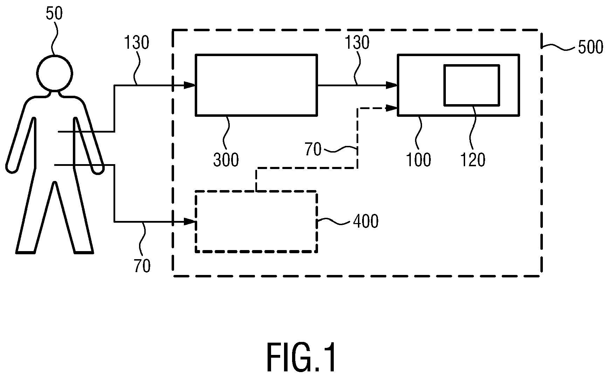

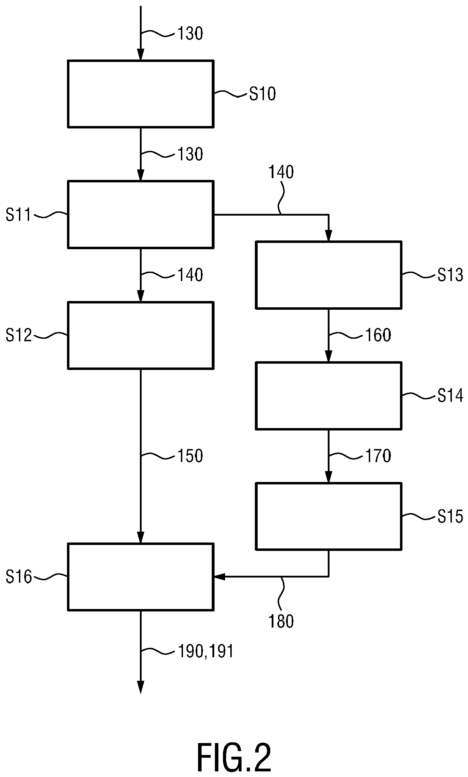

Fig. 1 shows a schematic diagram illustrating a system 500 and a device 100 for detecting atrial fibrillation of a subject 50, such as a patient, according to the present invention. The system 500 and the device 100 may be used, e.g., in hospitals, but also at home or in further health settings such as nursing or retirement homes. Fig. 2 shows a flowchart illustrating a method for detecting atrial fibrillation according to the present invention that may be executed by the device 100.

The system 500 comprises a sensor 300 for acquiring a cardiac activity signal 130 of the subject 50. Additionally, the system 500 may comprise another sensor 400 for measuring one or more other physiological signals 70 of the subject 50, such as the respiration rate (i.e., respiration frequency 170).

The sensor 300 may, e.g., be a photophletyhsmography (PPG) sensor, a ballistography (BCG) sensor or an electrocardiography (ECG) sensor. Several types of sensors, such as PPG sensors or ECG sensors, are capable of measuring both, respiratory frequency 170 and cardiac activity 130. Thus, the system 500 may also only have one sensor 300 for measuring the respiratory frequency 170 as well as the cardiac activity signal 130. The sensor 300 is preferably a wearable sensor that can be worn by the subject 50. Thus, no further cables limiting the freedom of the subject 50 are needed, which might hinder the workflow of a patient as well as a medical aide if the system 500 or the device is 100 used in clinical settings.

The (optional) second sensor 400 for measuring the respiratory frequency of the patient may, e.g., be an accelerometer, impedance sensor, resistive respiration belt, ballistocardiography sensor or a seismocardiography sensor.

The device 100 is configured to process the cardiac activity signal 130 (and the optionally provided) other physiological signal(s) 70 in order to reliably detect atrial fibrillation. For this purpose the device 100 preferably comprises a processing unit 120, which is configured to execute the steps of the method illustrated in Fig. 2.

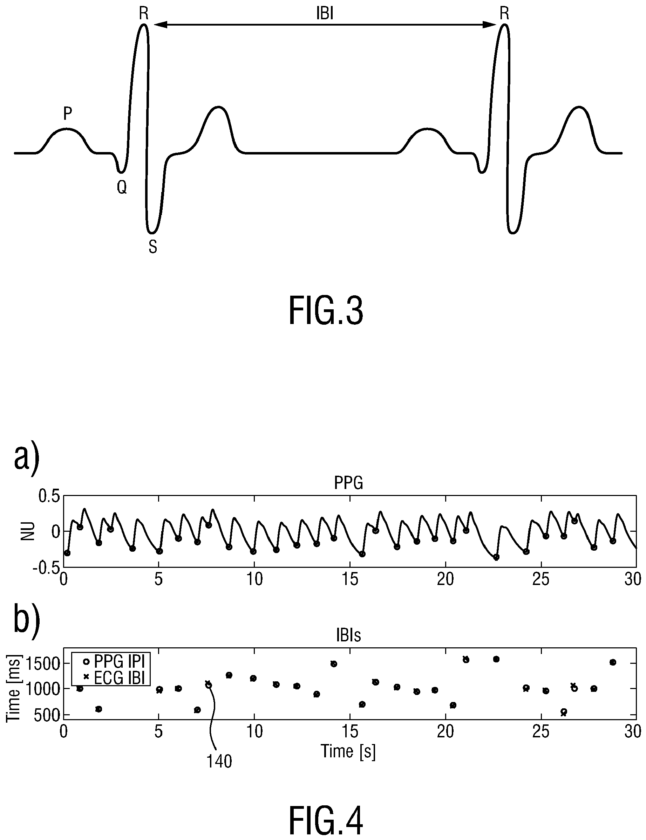

In a first step S10 the cardiac activity signal 130 is obtained (i.e. received or retrieved) from the sensor 300 to subsequently determine in step S11 an inter-beat interval (IBI) signal 140 representing the IBIs between heartbeats in said cardiac activity signal 130. The IBIs may be determined by detecting local minima and/or maxima in the cardiac activity signal 130 as illustrated in Fig. 3 showing an exemplary ECG signal. A typical ECG signal comprises various pulses, such as a P pulse being the atrial systole contraction pulse or the R pulse with a peak referring to ventricular contraction as shown in Fig. 3. The IBIs may be determined, e.g., as the time period between the maxima of two adjacent R pulses in an ECG signal.

Fig. 4(a) illustrates an exemplary signal waveform of a PPG signal recorded for a time period of 30 seconds. The PPG signal is an optical signal where the amplitude of the signal is directly proportional to pulse pressure and may be used as the cardiac activity signal 130 according to the present invention. The PPG signal for measuring cardiac rhythm or heart beat has a specific waveform profile with peaks and valleys that can be used to indicate the periodicity in heart rhythm and thus to extract the IBIs between heartbeats. In particular, the duration between the peaks of two adjacent pulses, or the valley of two adjacent pulses, is referred to the IBI.

The corresponding IBI signal 140 representing the IBIs between heartbeats in said PPG signal is shown in Fig. 4(b). As already explained above with reference to Fig. 3 the cardiac activity signal 130 may also be a ECG signal and Fig 4(b) shows that the extracted IBI signal of a PPG signal as well as the extracted IBI signal of a ECG signal recorded for the same time period and same patient results in the same data points for the IBI signal 140 indicating that using both ECG signal and PPG signal may be a viable option to be used as the cardiac activity signal 130.

Based on the IBI signal 140 the presence of atrial fibrillation of the subject is estimated (step S12). This may be achieved by analyzing the variability and/or chaotic structure of the IBI signal 140. Various models, such as a Markov algorithm model, may hereby be used to compute an initial probability 150 of atrial fibrillation from information on variability and chaotic structure of the temporal pattern of the IBI signal 140 to estimate the presence of atrial fibrillation. Further, a machine learning algorithm may be used to discriminate atrial fibrillation from other types of heart rhythm and to estimate the presence of atrial fibrillation.

In parallel, the power spectral density (PSD) spectrum 160 of the IBI signal 140 may be computed (step S13). According to an embodiment, this enables obtaining the respiratory frequency 170 of the subject (step S14). In particular, the respiratory frequency 170 may be obtained by deriving it from the computed PSD spectrum 160 as the frequency corresponding to a local maximum in the PSD spectrum 160 in the frequency range from 0.1 to 0.5 Hz, in particular from 0.15 to 0.4 Hz. Alternatively, the respiratory frequency and the corresponding respiratory frequency power may be determined by an analysis of PPG pulse envelope modulation, R-peak amplitude modulation in an ECG signal, bio-impedance modulation or analysis of chest movement using accelerometers.

According to another embodiment the respiratory frequency 170 may be obtained in a different way, e.g., it may be received or retrieved from the external sensor 400 as explained above with reference to Fig. 1.

The computed PSD spectrum 160 as well as the obtained respiratory frequency 170 of the subject are then used in step S15 to determine the respiratory frequency power 180 at said respiratory frequency 170 in the PSD spectrum 160. Based on the determined respiratory frequency power 180 determined in step S15 and the estimation 150 regarding the presence of atrial fibrillation determined in step S12 (in particular the initial probability 150) atrial fibrillation of the subject is finally detected in step S16 and a corresponding detection signal 190 may be issued. It is thus particularly determined if the subject shows indeed atrial fibrillation (i.e. the estimation in step S12 that there is atrial fibrillation has been correct) or if the subject does, in fact, not show atrial fibrillation (i.e. the estimation in step S12 that there is atrial fibrillation has been incorrect and the wrong estimation has been cause by respiration).

In an embodiment, a binary decision may be issued as final detection signal 190, said binary decision indicating the presence or absence of atrial fibrillation. This binary decision may be obtained by comparing the initial probability 150 of atrial fibrillation with a final probability 191, wherein the final probability 191 of the presence of atrial fibrillation may be determined in step S16 from the determined respiratory frequency power 180 and the initial probability 150.

Hence, according to the present invention the frequency pattern of the IBI signal 130 is evaluated in order to figure out the natural and benign forms of arrhythmia related to respiration and to reliably determine the presence or absence of atrial fibrillation.

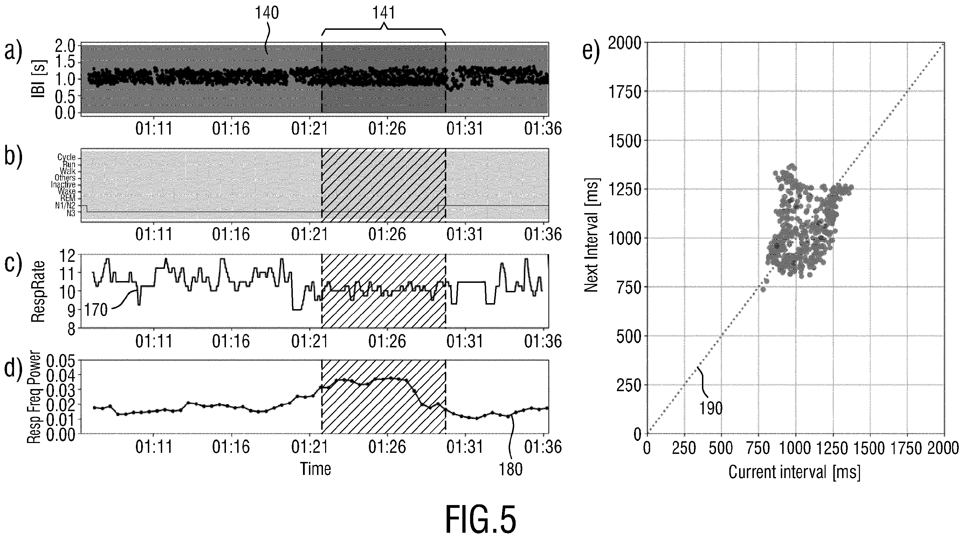

Figs. 5 to 8 show various signal diagrams used to further explain details and effects of the present invention.

Fig. 5 shows a diagram of an example of signals used in the detection of false atrial fibrillation of a subject experiencing normal sinus rhythm during sleep. This diagram illustrates why identifying variability features in an IBI signal 140 attributable to respiration allow reducing the number of false atrial fibrillation detection.

Fig. 5(a) represents an IBI signal 140 determined from a cardiac activity signal 130 which is recorded for a subject roughly for a time period of approximately 20 minutes. The IBI signal 140 represents the IBIs between heart beats in the cardiac activity signal 130.The area 141 in Fig. 5(a) indicates a time period during which (erroneously) a presence of atrial fibrillation is estimated (in step S16) due to a large initial probability 150 computed from the IBI signal 140, while during the rest of the time period absence of atrial fibrillation is estimated (in step S16).

Fig. 5(e) shows the corresponding Poincare plot of Fig. 5(a). A Poincare plot is used to quantify self-similarity in IBIs and visualizes the variability and chaotic structure in the IBI signal 140 shown in Fig. 5(a). The length of a next interval (next IBI) is plotted versus the current interval (current IBI). The dashed line 190 represents the case when the next IBI is constantly the same as the current IBI. The closer the points to the dashed line 190 through origin, the less variability and/or chaotic structure is in the IBI signal 140.

Fig. 5(b) shows a sleep state signal indicating that the subject is constantly in deep sleep states N1/N2 and N3 with a regular respiration rate. In particular, from 1:22 to 1:29 the respiration rate is very stable as shown Fig. 5(c) showing the respiratory frequency 170 (derived from the computed PSD spectrum 160 or obtained from the sensor 400).

Fig. 5(d) shows the respiratory frequency power 180 for the whole time period from 1:07 to 1:36. It is clearly shown there that the respiratory frequency power 180 is pronounced in the time period from 1:22 to 1:29. This information is used as an indication that the (erroneously) estimated presence of atrial fibrillation is due to the influence of respiration. In other words, according to the present invention, the problem of false detection of atrial fibrillation can be solved by detecting atrial fibrillation of the subject based on the determined respiratory frequency power and the estimated presence of atrial fibrillation relating to an initial probability of a presence of atrial fibrillation as illustrated in Fig. 2.

According to an embodiment, a binary decision 190 of the presence or absence of atrial fibrillation is generated, in particular by comparing the initial probability 150 of atrial fibrillation with a final probability 191. According to the example shown in Fig. 5 the binary decision would be 'no' for the time period 141, indicating that there is no atrial fibrillation and the that the estimation of the atrial fibrillation in step 12 has been wrong, caused by the strong influence of respiration in this time period 141.

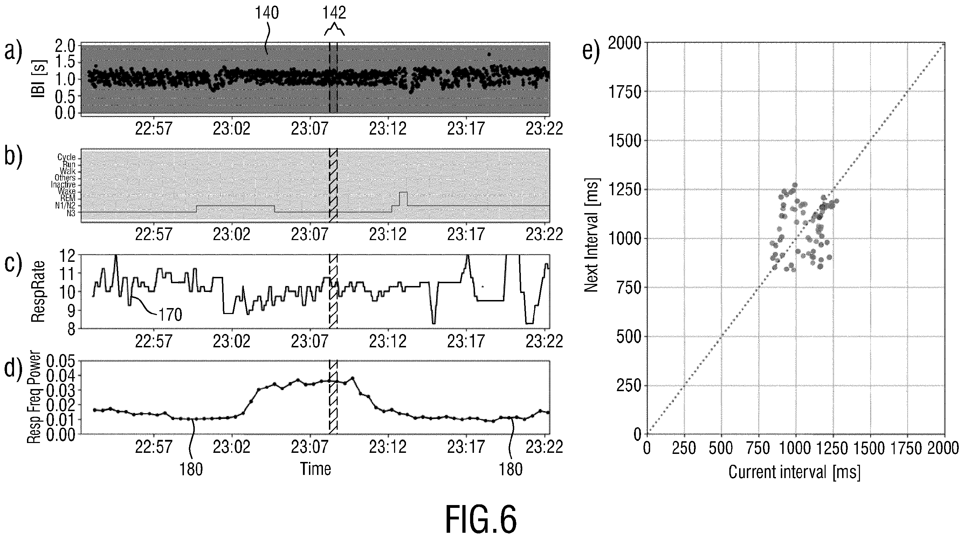

Fig. 6 shows a diagram of another example of signals used in the detection of false atrial fibrillation of a subject experiencing normal sinus rhythm during sleep. The subject is constantly in deep sleep states N1/N2, N3 or in REM sleep state as shown in Fig. 6(b). In particular, in the time period 142 from 23:04 to 23:12 the respiration rate 170 of the subject shows only slight variations and is stable as shown in Fig. 6(c). Fig. 6(d) shows that the respiratory frequency power 180 is large in the time period from 23:08 to 23:09 which falls into the time period where the respiration rate is stable as shown in Fig. 6(c). Again, it is clearly shown that the time period with large values of frequency respiration power 180 overlaps with the time period 142 in which atrial fibrillation has been estimated to be present due to a large initial probability 150 computed from the IBI signal 140 of Fig. 6(a).

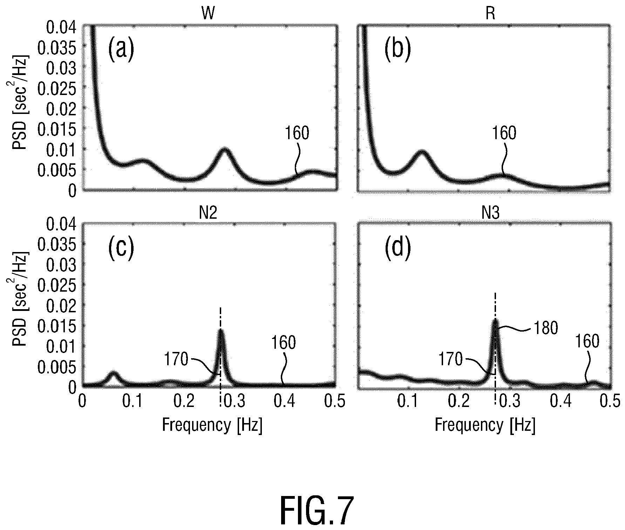

In particular, the deep sleep states N1, N2 and N3 may lead to erroneously detected atrial fibrillation due to a constant respiration rate. The PSD spectrum 160 in the absence of a regular respiration rate, such as during an awake (W) or during REM (R) sleep state, is shown in Figs. 7(a) and 7(b). The PSD spectrum 160 in the presence of a regular respiration rate, such as during the N2 or N3 sleep state, is shown in Figs. 7(c) and 7(d). The local maximum in the PSD spectrum at around 0.27 Hz is clearly pronounced in Figs. 7(c) and 7(d) and refers to the respiratory frequency 170, which is typically in a range from 0.1 to 0.5 Hz, in particular from 0.15 to 0.4 Hz. The value of the PSD in the respective PSD spectrum at the respiratory frequency 170 represents the respiratory frequency power 180.

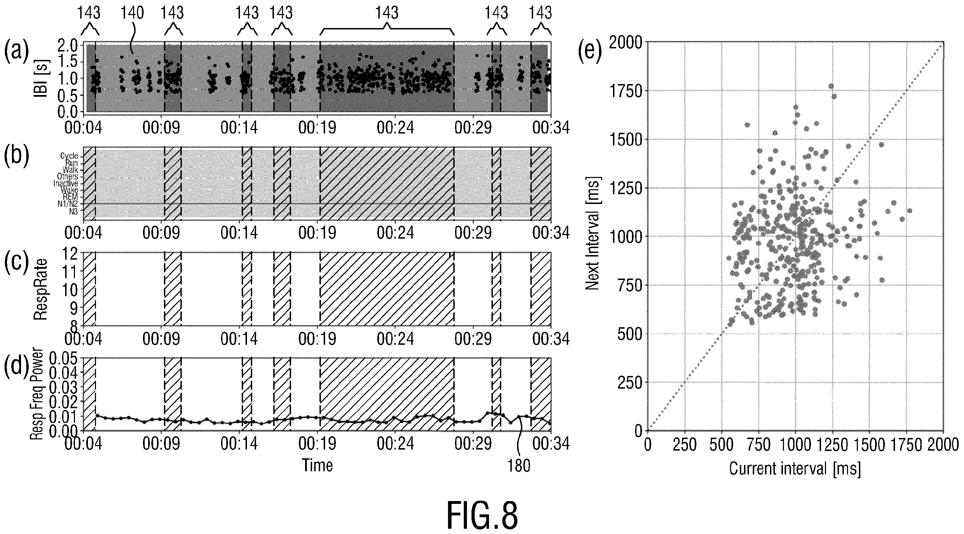

Fig. 8 shows a diagram of another example of signals used in the detection of correct atrial fibrillation of a subject experiencing atrial fibrillation during sleep. Fig. 8(a) shows several time periods 143 of atrial fibrillation detection. The corresponding Poincare plot is shown in Fig. 8(e). Said Fig. 8(e) visualizes that the IBI signal 140 has lots of variability and chaotic structure as many data points are far away from the dashed line through origin.

The subject is constantly in deep sleep state N1/N2 as shown in Fig. 8(b). Fig. 8(d) visualizes that the respiratory frequency power 180 is quite small and almost constant in the whole time period from 00:04 to 00:34 and thus shows that the atrial fibrillation estimation due to a large initial probability 150 in said time periods is not due to the influence of respiration, but atrial fibrillation has been correctly detected and is actually given. A binary decision 190 of the presence or absence of atrial fibrillation would hence be 'yes' according to the example shown in Fig. 8.

In the presence of atrial fibrillation reliable information of respiration rate (i.e., respiratory frequency 170) is often not possible. The PSD is, e.g., very low, and it is hard to figure out the respiratory frequency 170 and the respiratory frequency power 180 in the PSD spectrum 160. If this is the case, and if a presence of atrial fibrillation is estimated (in step S16) due to a large initial probability 150 computed from the IBI signal 140, the final probability 191 is large indicating the presence of atrial fibrillation. This situation is also visualized in Fig. 8, where Fig. 8(d) shows the maximum value of the PSD in the frequency range 0.2 - 0.6 Hz. Only when such maximum value is higher than the average PSD value in the frequency band the associated frequency is trusted as respiratory frequency. This is not the case in this situation so that the respiratory frequency cannot be derived from the corresponding PSD spectrum with sufficient reliability. For this reason Fig. 8(c) has been left blank, because even though a maximum value is found in the frequency range 0.2 - 0.6 Hz such maximum is not sufficiently higher than the others.

It shall be noted that each data point in Figs. 5(d), 6(d) and 8(d) refers to a local maximum in a PSD spectrum 160 as it is exemplarily shown for a subject in different awake and sleep states in Fig. 7. Thus, calculating the PSD spectrum 160 from the IBI signal 140 at each time point and extracting the PSD of the local maximum as shown in Fig. 6 enables calculating the respiratory frequency power (as shown in Figs. 5(d), 6(d) and 8(d)) from the PSD spectrum 160 and the respiratory frequency 170 (as shown in Figs. 5(c) and 6(c)).

The present invention thus enables to reliable detect atrial fibrillation and to reduce the probability of false atrial fibrillation detection using the IBI variability related to respiration rate and regularity. The present invention may be applied in the general ward in the hospital or the home. Other applications are other wards in the hospital and nursing or retirement homes. Patient monitoring and connected sensing applications may thus make use of the proposed atrial fibrillation detection.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Device (100) for detecting atrial fibrillation of a subject, said device (100) comprising a processing unit (120) configured to
obtain (S10) a cardiac activity signal (130) of the subject,
determine (S11) an inter-beat interval, IBI, signal (140) representing the IBIs between heart beats in said cardiac activity signal (130),
estimate (S12) the presence of atrial fibrillation of the subject based on the IBI signal,
obtain (S14) the respiratory frequency (170) of the subject,
determine (S15) the respiratory frequency power (180) at said respiratory frequency (170), and
detect (S16) atrial fibrillation of the subject based on the determined respiratory frequency power (180) and the estimated presence of atrial fibrillation.

2. Device according to claim 1, wherein the processing unit (120) is configured to receive or retrieve the respiratory frequency (170) from an external sensor (400) or to derive the respiratory frequency (170) and the corresponding respiratory frequency power (180) from a computed PSD spectrum (160) of the IBI signal (140).

3. Device according to any one of the preceding claims, wherein the processing unit (120) is configured to derive the respiratory frequency (170) from the computed PSD spectrum (160) as the frequency corresponding to a local maximum in the PSD spectrum (160) in the frequency range from 0.1 to 0.5 Hz, in particular from 0.15 to 0.4 Hz.

4. Device according to any one of the preceding claims, wherein the processing unit (120) is configured to determine the IBI signal (140) by detecting local minima and/or maxima in the cardiac activity signal (130).

5. Device according to any one of the preceding claims, wherein the processing unit (120) is configured to estimate the presence of atrial fibrillation by computing an initial probability (150) of the presence of atrial fibrillation from the IBI signal (140).

6. Device according to any one of the preceding claims, wherein the processing unit (120) is configured to detect atrial fibrillation by determining a final probability (191) of the presence of atrial fibrillation from the determined respiratory frequency power (180) and the estimated presence of atrial fibrillation.

7. Device according to claim 5 and 6, wherein the processing unit (120) is configured to generate a binary decision (190) of the presence or absence of atrial fibrillation, in particular by comparing the initial probability (150) with the final probability (191).

8. Device according to any one of claims 5 to 7, wherein the processing unit (120) is configured to compute the initial probability (150) from the IBI signal (140) by use of a Markov algorithm model or based on the variability and/or chaotic structure in the IBI signal (140).

9. Device according to claim 7, wherein the processing unit (120) is configured to compute the initial probability (150) from the IBI signal (140) by computing a root mean square of the successive differences, a normalized absolute deviation, a normalized absolute difference, a sample entropy, a Shannon entropy and/or coefficients of the sample entropy of the IBI signal (140) to analyze the variability and/or chaotic structure in said IBI signal (140).

10. Device according to claim 9, wherein the processing unit is configured to combine the analyzed variability and/or chaotic structure of the IBI signal with a machine learning algorithm to discriminate atrial fibrillation from other types of heart rhythm.

11. Device according to claim 10, wherein the machine learning algorithm is a support vector machine model or logistic regression model.

12. System (500) for detecting atrial fibrillation of a subject, said system (500) comprising:
a sensor (300) configured to acquire a cardiac activity signal (130) of the subject; and
a device (100) according to any one of the preceding claims for detecting atrial fibrillation of a subject based on the acquired cardiac activity signal (130) of the subject.

13. System according to claim 12, wherein the sensor (300) is a photoplethysmography sensor, an electrocardiography sensor, a ballistocardiography sensor, or another wearable sensor configured to continuously record cardiac activity signals (130) of the subject.

14. Method for detecting atrial fibrillation of a subject, said method comprising the steps of:
obtaining (S10) a cardiac activity signal (130) of the subject,
determining (S11) an inter-beat interval, IBI, signal (140) representing the IBIs between heart beats in said cardiac activity signal (130),
estimating (S12) the presence of atrial fibrillation of the subject based on the IBI signal (140),
obtaining (S14) the respiratory frequency (170) of the subject,
determining (S15) the respiratory frequency power (180) at said respiratory frequency (170), and
detecting (S16) atrial fibrillation of the subject based on the determined respiratory frequency power (180) and the estimated presence of atrial fibrillation.

15. Computer program comprising program code means for causing a computer to carry out the steps of the method as claimed in claim 14 when said computer program is carried out on the computer.
